# EUROPEAN PATENT SPECIFICATION

(11) **EP 0 724 884 B1**
(45) Date of publication and mention of the grant of the patent: **02.05.2002**
(21) Application number: 92923166.0
(22) Date of filing: 05.11.1992
(51) Int. Cl.: A61K 38/18

(54) **SIDE EFFECT INHIBITOR FOR CANCER THERAPY**
INHIBITOR VON NEBENWIRKUNGEN ZUR KREBSTHERAPY
INHIBITEUR DES EFFETS SECONDAIRES DANS LA THERAPIE DU CANCER

(30) Priority: 07.11.1991 JP 32141291
(43) Date of publication of application: 07.08.1996
(73) Proprietor: Nakamura, Toshikazu, Higashi-ku, Fukuoka-shi 813 (JP)
(72) Inventor: Nakamura, Toshikazu, Higashi-ku, Fukuoka-shi 813 (JP)
(74) Representative: von Kreisler, Alek, Dipl.-Chem.
(86) International application number: JP9201433
(87) International publication number: WO9308821

(56) References cited:
- JP-A- 1 143 900
- JP-A- 2 134 323
- JP-A- 3 072 883
- JP-A- 3 130 091
- JP-A- 3 204 899
- JP-A- 60 045 534
- JP-A- 63 022 526
- CELL BIOLOGY INTERNATIONAL REPORTS, vol. 15, no. 5, May 1991 pages 397-407, SHIMA N. ET AL.
- FEBS LETTERS, vol. 291, no. 2, October 1991 AMSTERDAM NL, pages 229-232, TAJIMA H. ET AL.

## Description

### Technical Field

This invention is concerned with the use of hepatocyte growth factor (HGF, hereinafter) for preparing a medicament for reducing or preventing side effects induced by cancer therapy.

### Background Art

The development of a new cancer therapy or a new anti-cancer drug is the greatest concern for medical-treatment researchers as well as for workers in the field of medical pharmaceuticals; it is also the greatest problem in medicine at present.

As the therapy for cancer, the conventional method, that is, the administration of anti-cancer drugs such as alkylating agents, metabolic competitors, antibiotics, hormones and modifying agents of biological reactions, administration of radiation, and surgical treatments, has been carried out alone or in combination.

The conventional cancer therapy has chiefly been aimed at the removal and obliteration of cancerous lesions, but the recognition has been made that it is important to carry out therapies under consideration of the total improvement of the biological functions in the patient, with special emphasis on reducing side effects. From this viewpoint, cancer therapy as a whole is being reexamined. Since very little qualitative difference is recognized between a cancer cell and a normal cell by conventional anti-cancer drugs used thus far in chemotherapy, severe side effects have been induced by anti-cancer drugs with high anti-cancer effectiveness, so much so that the statement was made that an anti-cancer drug without side effects is not effective in cancer therapy. Since anti-cancer agents at present are inevitably accompanied by toxicity, the chemotherapy must utilize the effectiveness of the anti-cancer agent to the maximum while the toxicity is reduced as much as possible. In radiation therapy, the body is exposed to radiation, which is inherently deleterious, resulting in side effects of various types. Here, it is also extremely important to reduce the side effects.

Thus, in chemotherapy and radiation therapy for cancer, normal cells and tissues are affected in addition to cancer cells, resulting in the side effects of hematopoietic depression, nausea and vomiting, heart damage, lung fibrosis, liver damage, kidney damage, hair loss, and skin symptoms. More severe damage is likely to be inflicted in cells and tissues with a high proliferation rate. Because of such side effects, more potent therapy cannot be performed, presenting a bottleneck in cancer therapy.

With these problems, research has been carried out on decreasing the toxicity of anti-cancer agents on the normal cells and tissues. As a result, γ-globulin, cytochrome C, adenine, SH compounds, and the vitamin-B group have been used as inhibitors of side effects. However, their effectiveness is not sufficient.

Recently, colony stimulating factor (CSF) was used to reduce the toxicity of an anti-cancer treatment on the hematopoietic tissues, with amelioration of leukopenia under such an anti-cancer treatment having been reported (Motoyosi, K. et al., Exp. Hematol;, 14:1069-1075, 1986). CSF has been known to be effective in reducing damage in the cells and tissues derived from the mesoderm, but there is no substance known to reduce the side effects on cells and tissues derived from the ectoderm and endoderm. Much demand has been expressed for such a substance.

This invention is offered to solve the aforementioned problems. The aim of this invention is to offer an agent capable of preventing side effects induced by cancer therapy by amelioration of the damage suffered by normal cells and tissues.

### DISCLOSURE OF THE INVENTION

This invention relates to the use of hepatocyte growth factor (HGF) for preparing a medicament for preventing side effects of cancer therapy in humans or other mammals.

After intensive study of a growth factor of hepatocytes for a long time, the inventor succeeded in the isolation and purification of HGF. The inventor demonstrated that HGF acted as a growth factor, not only on the hepatocytes but also on epithelial cells in general, with several inventions having been completed. The inventor described the development and application of HGF as a drug for renal diseases after the discovery that HGF promoted proliferation of the proximal tubule cells of the kidney in Japanese Patent Application No. 158841/1990. The inventor succeeded in applying and developing HGF as the drug for wound healing and for the treatment of skin ulcers or as the drug for the proliferation of hair root cells after the discovery that HGF accelerated the proliferation of normal epithelial cells such as melanocytes and keratinocytes. The detailed explanation of a patent application was made in this aspect in Japanese Patent Application No. 419158/1990. It must be emphasized that HGF has a practical value in cancer therapy in that it does not have carcinogenic activity or the activity of accelerating the proliferation of cancer cells, which are observed with many growth factors such as EGF. The inventor also demonstrated that HGF could be used as an anti-cancer agent utilizing the property of inhibiting cancer cell proliferation as observed in the HepG2 cell line derived from a human hepatoma and the IM9 cell line derived from a lymphoblast tumor in Japanese Patent Application No. 140812/1991. After the discovery that HGF reduced the damage suffered by normal cells and tissues under cancer therapy, resulting in amelioration of the side effects in the continuing research on HGF, the inventor completed this invention.

### Brief Description of Drawings

Figure 1 shows the amino acid sequence of the precursor of human HGF.

Figure 2 shows the base sequence of the gene coding the amino acid sequence (Figure 1) of the precursor of human HGF.

Figure 3 shows the time course of the leakage of GOT from the hepatocytes exposed to carbon tetrachloride. In the figure, the symbol O indicates the culture without such an addition (control), the symbol ● indicates the culture with the addition of carbon tetrachloride, and the symbol □ indicates the culture with the addition of carbon tetrachloride and HGF (refer to Test Example 1).

Figure 4 shows the inhibitory effect of HGF (dose-response curve) on the leakage of GOT from the hepatocytes exposed to carbon tetrachloride (refer to Test Example 2).

Figure 5 shows the inhibtory effect of HGF on GOT leakage from the hepatocytes exposed to carbon tetrachloride (refer to Test Example 3).

Figure 6 shows the activity of rat HGF for acceleration of the proliferation in rat proximal tubule cells (refer to Test Example 5). In the figure, the symbol ● indicates the culture with the addition of HGF and the symbol □ indicates the culture with the addition of EGF and insulin (positive control).

Figure 7 shows the activity of HGF for acceleration of the proliferation (number of cells) in normal human skin cells (melanocytes) (refer to Test Example 6).

Figure 8 shows the activity of HGF for acceleration of proliferation (synthesis of replication DNA) in the normal human skin cells (melanocytes) (refer to Test Example 7).

Figure 9 shows the activity of HGF for acceleration of the proliferation (number of cells) in normal human skin cells (keratinocytes) (refer to Test Example 8).

Figure 10 shows the activity of HGF for acceleration of the proliferation (synthesis of replication DNA) in normal human skin cells (keratinocytes) (refer to Test Example 9).

### Best Mode for Carrying Out the Invention

In the medicament for preventing side effects of cancer therapy of the invention, the effective component is HGF, which was discovered by the inventor in the serum of rats with a regenerating liver as a protein factor having the capability of inducing proliferation in mature hepatocytes in vitro (Biochem. Biophys. Res. Commun., 122:1450, 1984). The inventor further succeeded in isolating HGF from rat platelets and in determining a part of its amino acid sequence (FEBS Letter, 22:311, 1987). Furthermore, the inventor carried out cDNA cloning for HGF derived from humans and rats based on the HGF amino acid sequence clarified, and succeeded in obtaining HGF as a pure protein by recombination of the cDNA in animal cells (human HGF: Nature, 342:440, 1989; rat HGF: Proc. Natl. Acad. Sci., 87:3200, 1990).

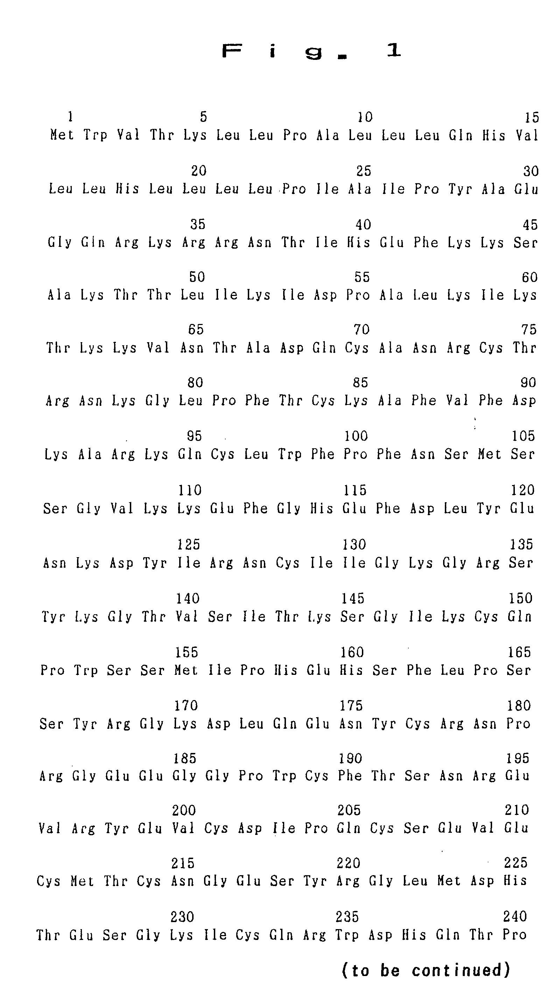

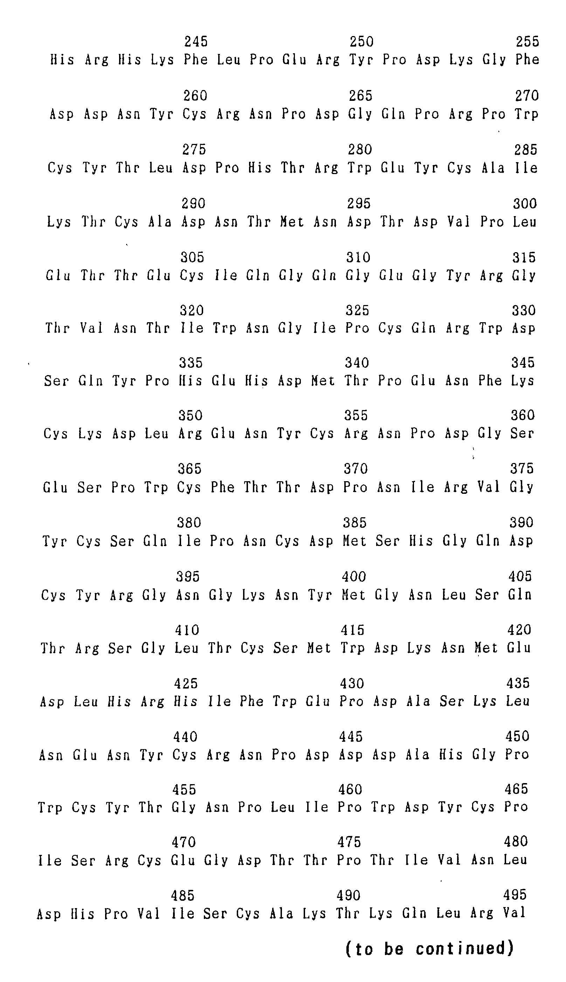

The molecular weight of the aforementioned HGF was determined to be 82-85 kD from SDS polyacrylamide gel electrophoresis. Rat HGF has a heterodimer structure in which an α chain with 463 amino acids is crosslinked by one disulfide bond to a β chain with 233 amino acids. There are 2 glucosamine sugar-binding portions in both the α chain and the β chain. Human HGF, with the same physiological activity, is composed of an α chain with 463 amino acids and a β chain with 234 amino acids. Four Kringle structures are present, similar to the fibrinolytic enzyme plasmin, in the α chain, and the amino acid sequence of the β chain have a homology of about 37% with the β chain of plasmin, having serine protease activity. Figure 1 and Figure 2 show the amino acid sequence of the human HGF precursor and the base sequence of the gene coding it, respectively. Human HGF is synthesized as the precursor with 728 amino acids, as shown in Figure 1, and is then divided into an α chain containing 463 amino acids (from Gln at the 32nd position to Arg at the 494th position in the sequence of Figure 1) and a β chain containing 234 amino acids (from Val at the 495th position to Ser at the 728th position in Figure 1). The homology in the amino acid sequence between rat HGF and human HGF is 91.6% in the α chain and 88.9% in the β chain and the activity can be utterly exchangeable.

The aforementioned HGF can be obtained by various methods. For examples, HGF can be extracted and purified from organs such as the liver, spleen, lung, bone-marrow, brain, kidney, and placenta, as well as blood cells such as platelets and white blood cells, and the plasma or serum of mammals such as the rat and cow. It is possible to carry out the primary culture of cells producing HGF or the culture of a cell line with such productivity, and to separate and purify HGF from the cultured product. It is possible to recombine the gene coding HGF with a proper vecter by a genetic engineering method and to insert the recombinant gene into a proper host cell, followed by culture of the transformed host cell from which the desired recombinant HGF is obtained (Nature, 342:440, 1989). There is no special restriction on the type of the aforementioned host cell; one can use various host cells such as E. Coli, B. subtilis, yeasts, filamentous fungi, or plant or animal cells that have been used in the genetic engineering methods in the past.

In a concrete example of extracting and purifying HGF from'tissues, carbon tetrachloride is intraperitoneally injected into rats as shown in Reference Example 1, followed by extirpation and homogenization of a liver afflicted by hepatitis. The supernatant of the homogenized liver is treated with gel column chromatography for purification using S-sepharose® and heparin sepharose®. As shown in Reference Example 2, a method of genetic engineering is used in that the gene coding the amino acid sequence of HGF shown in Figure 2 is recombined with a vector such as bovine papilloma virus NDA to obtain an expression vector, which is used for the transformation of animal cells such as Chinese hamster ovary cells (CHO), mouse C127 cells, or monkey COS cells, to obtain HGF from the supernatants of their cultured fluid.

In the HGFs thus obtained, there are possibilities that a part of the amino acid sequence will be missing or replaced with other amino acids, that another amino acid sequence is partly inserted, that 1, 2, or more amino acids are attached to the C or N terminals, or that a sugar is missing or replaced with others. Such a slight modification of HGF could be accepted. As such, one can cite substances described in Japanese Kokai Patent No. 130091/1991 and W090/10651. These substances can be used in this invention and they are within the scope of this invention.

One can use HGF as the effective component of the drug for the prevention of side effects by cancer therapy, derived from mammalian species including humans, cows, horses, rats, and rabbits. These HGFs show an excellent preventive influence on side effects in the cancer treatment of mammals. In other words, the drug of this invention can be used not only in humans but also in other animals.

It is possible to use the drug for the prevention of side effects as a therapeutic agent for cancer, together with the chemotherapy using anti-cancer agents and radiation therapy. Since HGF accelerates cell proliferation in the hepatocytes and the proximal tubule cells of the kidney, amelioration of the damage is performed in the liver and kidney by HGF. Also, HGF has the activity of promoting proliferation of the epithelial cells in general, so that side effects such as nausea, vomiting, and hair loss can be reduced. Such as ameliorating is due to the fact that nausea and vomiting are induced by the damage to the epithelial cells of the digestive canal resulting in stimulation of the peripheral nerve nearly, and that hair loss is induced by the damage suffered by the tissue with a high proliferation rate near the hair follicles. HGF exerts a selective activity on promoting proliferation of cells and tissues of ectodermal and endodermal origin, such as epithelial cells, melanocytes and keratinocytes. Therefore, deleterious effects of the anti-cancer agent and radiation are reduced with regard to these cells and tissues. As a result, still more potent cancer therapy can be applied, having an improved effect of the cancer treatment.

The cells treated by HGF are more resistant to cellular damage, with HFG administration being effective as a pretreatment before chemotherapy and radiation therapy for the prevention of side effects in the future.

The important point of HGF as a mediacal pharmaceutical agent is that HGF exerts the promotion of proliferation in the cells at the Gl stage, or in the first stage of proliferation, and not on the cells at the G0 stage or the resting stage. This property implies the HGF has no effect at all on tissues without damage, and only promotes proliferation and regeneration of the damaged tissues. Consequently, even if a high dose of HGF is administered or if HGF is brought to healthy tissue without damage via blood, carcinogenic activity is not induced and excessive proliferation is not promoted in the normal tissues. The medicament for preventing side effects of cancer therapy of this invention can take various forms of pharmaceutical products such as a liquid, solid product, and capsules. In general, the injection fluid is prepared with HGF alone or in combination with a common carrier, with a preparation for external application being produced with HGF together with the commonly used carrier. The preparation for injection can be prepared using a common method. For example, after dissolution in a proper solvent such as sterile water, buffer, or physiological saline, the solution of HGF is sterilized by filtration and sterile containers are filled with the sterilized HGF solution. The concentration of HGF in the injection fluid is usually adjusted at 0.0002-0.2 (W/V%), preferably at 0.001-0.1 (W/V%). As the drug for external application, pharmaceutical products are formed as a gel, ointment, or liquid. The HGF content therein is properly adjusted, depending on the disease to be treated and the location of application.

In the preparation of the pharmaceutical product, it is preferable to add a stabilizer such as albumin, globulin, gelatin, mannitol, glucose, dextran, and ethylene glycol. The pharmaceutical product can contain additives necessary for production, such as an excipient, a dissolving aid, an antioxidant, a pain-alleviating agent, and an agent for isotonicity. In the case of the product in the form of a solution, it is preferable to store it under frozen conditions or after the removal of water by a process such as freeze-drying.

The drug of this invention for the prevention of side effects is administered through a proper route that is adequate for the form of the pharmaceutical product. For example, the product for injection can be administered by intravenous, intraarterial, subcutaneous, or intramuscular injection. The dosage must be adjusted according to the symptoms of the patient, age, and body weight. The usual dosage of HGF is 0.01-100 mg administered once or several times per day.

The present invention will then be described in more detail referring to Reference Examples, Test Examples and Examples.

### Reference Example 1

### Isolation of HGF from rat liver

Carbon tetrachloride (0.2% body weight of rat) was intraperitoneally administered to Wister rat, and 30 hours later, the rat liver was removed. The liver was homogenized by a waring blender, after which it was centrifuged at 10,000 rpm for 20 minutes with a Hitachi 20 PR-52 cooling centrifuge to give a supernatant. The supernatant was dialyzed against a mixed solution of 50 mM tris hydrochloric acid buffer (pH 8.5), 0.15 M NaCl, 10 mM HEPES, 2 mM CaCl₂ and 0.01% Tween® 80 at 4°C for a whole day. The dialyzed solution was poured onto an S-Sepharose® (FF) (Pharmacia Co.) column equilibrated with dialyzing fluid, and after washing, it was eluted with the gradient of NaCl. HGF was eluted at about 0.7 M NaCl concentration. This HGF was then purified by Blue Tris acryl M (IBF Co.) chromatography. Elution was conducted with the gradient of arginine, and HGF was eluted at about 0.25 M arginine concentration. The obtained fraction was then purified by heparin-Sepharose® (Pharmacia Co.) chromatography. Elution was conducted with the gradient of NaCl, and HGF was eluted at about 1 M NaCl concentration, which was then purified by phenyl 5PW (Toso Co.) chromatography. Elution was conducted with the decrease gradient of NaCl and the increase gradient of ethylene glycol, and 10 µg of HGF was obtained from livers of 100 rats. Relative activity of HGF was 500,000 unit/mg. To the obtained HGF was added 0.25% BSA (bovine serum albumin) and the mixture was dialyzed with PBS (phosphate buffer saline).

### Reference Example 2

### Production of HGF by gene recombination method

Using gene recombination method, human cell-derived HGF was produced.

In accordance with the Wigler et al method (Cell, 11, 223, 1977), Chinese hamster ovary (CHO) cells transformed with a gene coding for the amino acid sequence of the human HGF were cultured, and human HGF was obtained from the culture supernatant thereof. That is, a cDNA library prepared from mRNA of human liver was subjected to screening, by which clone HAC19 and clone HBC25 coding for the amino acid sequence of the human HGF were obtained.

DNAs from the HAC19 and the HBC25 were digested with BamHI and ScaI, and ScaI and PstI, respectively. The thus-obtained two DNA fragments were ligated with Blue Script KSII at BamHI and PstI sites to obtain pBS[hHGFII]. The pBS[hHGFII] was digested with XbaI, SalI and NaeI, and given blunt ends by T4 DNA polymerase, after which an about 3 Kb DNA fragment coding for the human HGF was inserted at EcoRV site of an expression vector pBPMT prepared with bovine papilloma virus DNA as a vector, to give pBPMT[hHGFII]. The thus-obtained HGF expression vector pBPMT[hHGFII] was used to transform CHO cells by the DEAE dextran method. Selection of the transformants was conducted by growth thereof in a medium containing G418. The cell line BPH89 showing high HGF producing activity was selected from among the transformants obtained. After the BPH89 cells were grown in a medium supplemented with fetal calf serum, the medium was exchanged every 2 days, followed by purification according to a modification of the purification method as described in Reference Example 1.

### Reference Example 3

### Isolation of hepatocytes from adult rats and primary culture

Hepatocytes of adult rats were isolated by perfusion of the liver using collagen according to the method of Seglen (Meth. Cell Biol. 13:29-33, 1976). The primary culture was carried out as follows: Isolated hepatocytes were dispersed in William's medium (5% bovine serum, 10⁻⁹ M insulin and 10⁻⁹ M dexamethasone) and the cells were seeded in a 12-well plastic dish (Corning Co.) having the bottom coated with Type-I collagen. The culture medium was exchanged with the medium containing aprotinin at 0.5 µg/ml and without serum and hormones 2 hours later.

The protective effect of HGF which is the effective component of the side effect inhibitor for cancer therapy of the invention, on the hepatocytes is shown in Test Examples 1-4 described below.

The protective effect of HGF on the hepatocytes was tested by the effect on the leakage to enzymes from the cytoplasm of the hepatocytes in the primary culture. Carbon tetrachloride is a poison acting on the liver and induces hepatitis in the living body. The administration of carbon tetrachloride to experimental animals induces the leakage to glutamate-oxaloacetate transferase (GOT), glutamate-pyruvate aminotransferase (GPT), and lactate dehydrogenase (LDH), as the cytoplasmic enzymes in a dose-dependent manner, into the blood.

In order to mimick this phenomenon, hepatocyte primary-culture added with the liver poison was used. Thus the leakage of cytoplasmic enzymes such as GOT was measured in this culture system, and the protective activity on the hepatocytes by HGF was estimated along with measurement of the degree of inhibition of leakage of GOT or others.

In the following Test Examples 1-4, recombinant human HGF after preparation as described for HGF in Reference Example 2 was used. As the carbon tetrachloride solution, carbon tetrachloride was dissolved in DMF at the concentration of 1 M; this solution was diluted with William's medium to the final concentration of 5 mM.

### Test Example 1

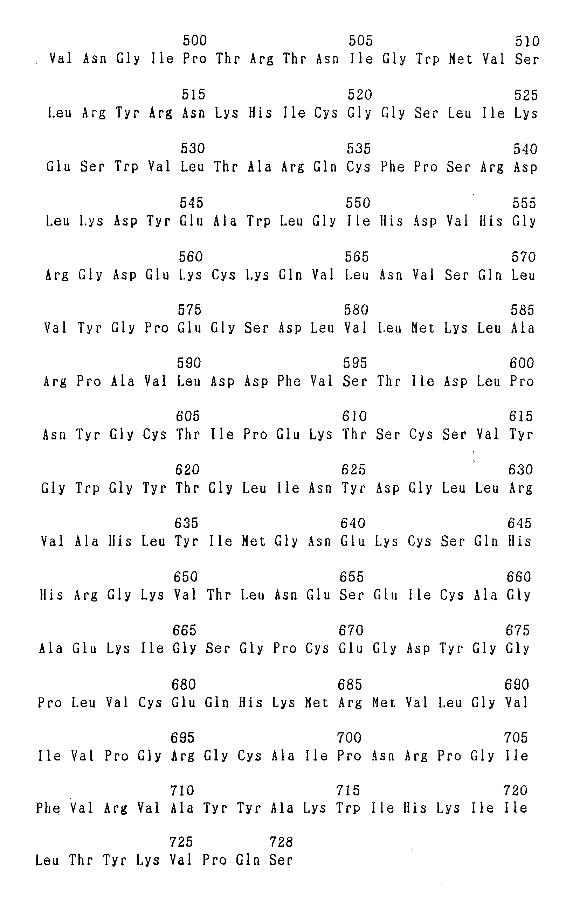

The primary-culture hepatocytes described in Reference Example 3 were seeded at the surface density of 1.2 x 10⁵ cells/cm² and on the next day, the medium was changed to one without serum, followed by the addition of HGF at 10 ng/ml and/or carbon tetrachloride at 5 mM. After culture for a preset time, the media were collected and centrifuged to obtain a supernatant. The GOT activity in the supernatant was measured by the conventional method. The result is shown in Fig. 3 (average of data from 3 wells). In the figure, the following symbols are used: O: culture without such addition (control), ●: culture with the addition of carbon tetrachloride, and □: culture added with carbon tetrachloride and HGF.

As shown in Fig. 3, in the culture added with carbon tetrachloride, GOT leaked into the medium, indicating damage to the hepatocytes. On the other hand, in the culture with HGF in addition to carbon tetrachloride, the leakage of GOT was at the same level as the control, indicating the inhibition of damage to the hepatocytes.

The LDH activity and GPT activity in the supernatant described above were also measured and it was confirmed that the leakage from hepatocytes of these enzymes was also inhibited by HGF presence.

### Test Example 2

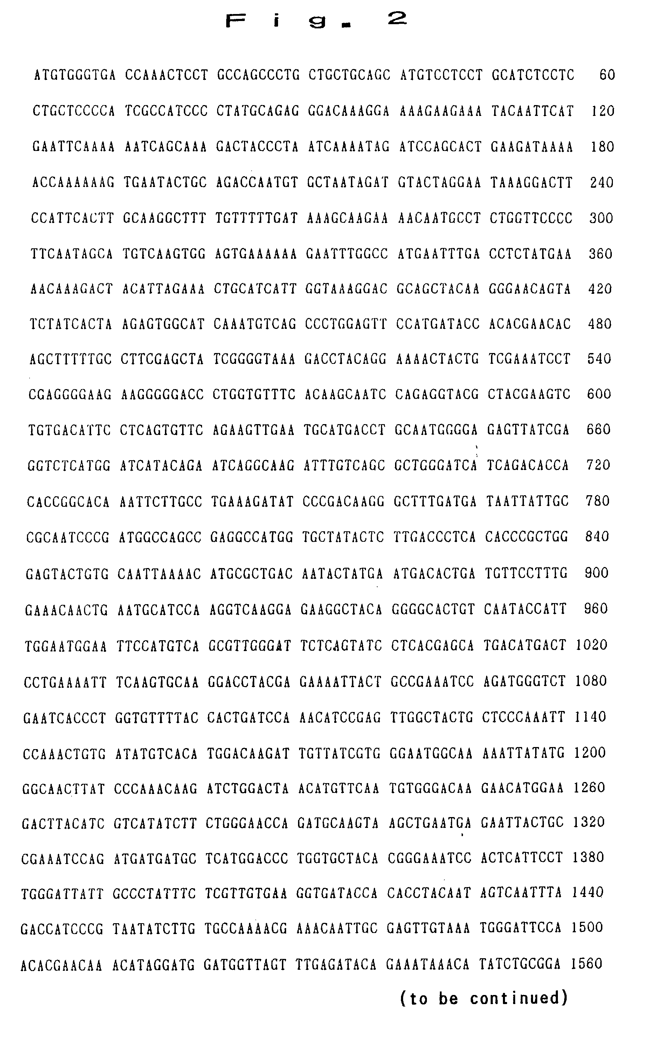

The primary-culture hepatocytes described in Reference Example 3 were seeded at the surface density of 1.2 x 10⁵ cells/cm² and on the next day, the medium was changed to one without serum. Carbon tetrachloride was added at the final concentration of 3 mM and HGF was further added at various concentrations. After 24 hours' incubation, the media were collected and centrifuged to obtain a supernatant. The GOT activity in the supernatant was measured by the conventional method. The result is shown in Fig. 4 (average of data from 3 wells). The GOT activity was 6.0 mU/ml in the control culture (without the addition of carbon tetrachloride) after 24 hours' incubation.

As shown in Fig. 4, the leakage of GOT from the hepatocytes was inhibited by HGF, in a dose-dependent manner, in the range of 0-8 ng/ml, indicating the protective activity of HGF on the hepatocytes.

### Test Example 3

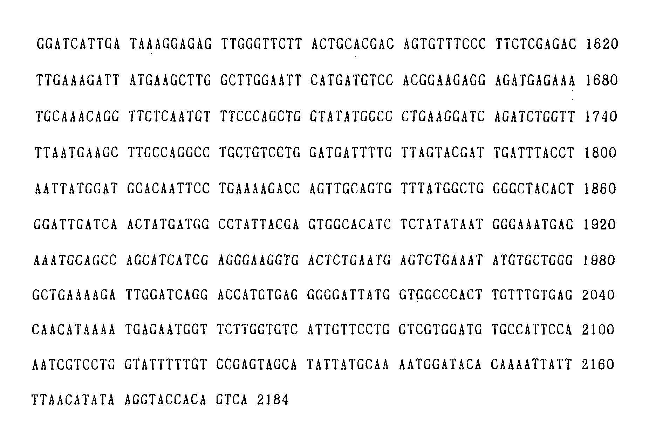

The primary-culture hepatocytes described in Reference Example 3 were seeded at the surface density of 1.2 x 10⁵ cells/cm² and cells were exposed to HGF (10 ng/ml) or carbon tetrachloride (5 mM) for a preset time. On the next day, the medium was changed to one without serum, followed by the addition of HGF (10 ng/ml) and/or carbon tetrachloride (5 mM). After 24 hours' incubation, the media were collected, followed by centrifugation to obtain a supernatant. The GOT activity in the supernatant was measured by the conventional method. Fig. 5 shows the result (average of data from 3 wells). In the figure, 0 hour indicates the time for the exchange of the culture medium. White bars indicate the time for exposure of the cells to carbon tetrachloride and stippled bars indicate the time for exposure of the cells to HGF.

As shown in Fig. 5, the leakage of GOT was inhibited by pretreatment of the cells with HGF before exposure to carbon tetrachloride. Also, the leakage of GOT was inhibited by treatment of the cells with HGF previously exposed to carbon tetrachloride.

### Test Example 4

The primary-culture hepatocytes described in Reference Example 3 were seeded at the surface density of 1.2 x 10⁵ cells/cm² and the medium was changed to one without serum on the next day, followed by the addition of HGF (10 ng/ml) and carbon tetrachloride (5 mM) or mitomycin C (8 µM). After culture for 24 hours, the media were collected and centrifuged to obtain a supernatant. The GOT activity in the supernatant was measured by the conventional method. Table I shows the results (average of data from 3 wells).

As shown in Table I, HGF inhibited the leakage of GOT induced by liver poisons, carbon tetrachloride, and mitomycin C.

**Table I**

| Liver poison | GOT activity (mU/ml) | |
|---|---|---|
| | No addition | 10ng/ml HGF |
| Control (0.5% DMSO) | 6.0 ± 0.4 | 4.8 ± 0.2 |
| Carbon tetrachloride (5 mM) | 12.7 ± 1.2 | 4.9 ± 0.2 |
| Mitomycin C (8 µM) | 13.5 ± 1.3 | 5.2 ± 0.3 |

### Test Example 5

### Effects of HGF on promotion of proliferation of proximal tubule cells

The growth activity of the HGF on proximal tubular cells was confirmed as follows.

### ① Isolation of rat kidney proximal tubular cells

Wister rat was anesthetized with a barbital anesthetic and its abdomen was incised to remove kidney on an ice-cooled plate.

Renal cortex was collected and cut in small pieces, which was then subjected to Down's homogenizer. Homogenate obtained was filtered through a 245 µm nylon mesh, and further filtered through a 105 µm nylon mesh to separate renal tubular cells and granular cells, after which the fraction left on the mesh was transferred to an ice-cooled Eagle's minimum essential medium. Since a small amount of granular cells was left in this fraction, 0.01% collagenase was added to the medium, and the medium was treated at 37°C for 3 minutes, followed by removal of fibroblasts and centrifugation at 80 g for 2 minutes to give purified proximal tubular cells.

### ② Growth activity of HGF on proximal tubular cell proliferation

HGF was added to the culture system of the proximal tubular cells of kidney isolated as described above, and cell growth-promoting activity was examined in terms of increased DNA synthesis. That is, proximal tubular cells as obtained above were suspended in DME·F-12 mixed medium (DEM medium: ham F-12 medium=1:1, Nissui Seiyaku Co., Japan) supplemented with 1x10⁻⁸ M insulin (Sigma Co., USA), 1x10⁻⁸ M dexamethazone (Wako Junyaku Co., Japan), 5 µg/ml transferrin (Sigma Co., USA) and 5 U/ml aprotinin (Mochida Seiyaku Co., Japan) and seeded into a 24-well multiplate at a concentration of 4 x 10⁴ cells/well. After 24 hours' incubation at 37°C in the presence of 5% CO₂, 30% O₂ and 65% N₂, the medium was changed to DME·F-12 mixed medium supplemented with 5 U/ml aprotinin, followed by 48 hours' incubation under the same incubation conditions. With the exchange of the medium to newly-prepared DME·F-12 mixed medium supplemented with 5 U/ml aprotinin, rat HGF as obtained in Reference Example 1 (added with 0.25% BSA and dialyzed with PBS) was added as a test sample and also, a given amount of 10 ng/ml EGF (epithelial cell growth factor, derived from male mouse submandibular gland) + 1 x 10⁻⁷ M insulin was added as positive control. After incubation for 16 hours, 1 µCi/ml of ¹²⁵I-deoxyuridine (New England Nuclear Co., USA) was added at 10 µl/well. Four hours later, cells were washed with PBS, placed in a 10% trichloroacetic acid solution and incubated for 5 minutes.

The trichloroacetic acid was removed, cells were subjected to lysis with 1 M sodium hydroxide solution and radioactivity was measured by a gamma counter.

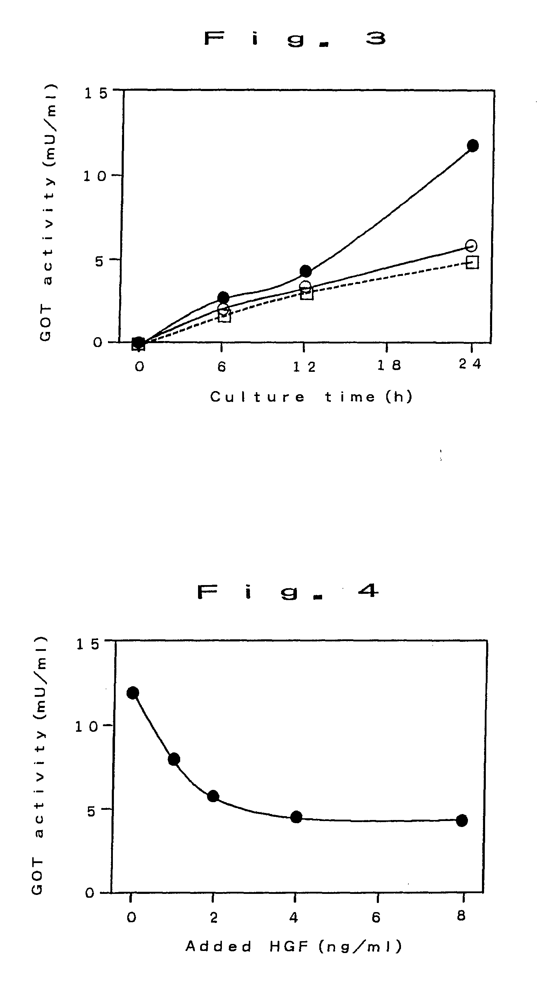

The results are shown in Fig. 6. As shown in Fig. 6, rat HGF showed the activity of increasing the proliferation rate of proximal tubule cells of the rat kidney in a dose-dependent manner. By the addition of HGF at 2 ng/ml, the DNA synthesis of the cultured cells was increased to about 2 times, and to about 3 times by the addition of HGF at 10 ng/ml. The results indicated the activity of HGF, as the effective component of this invention, in inducing the proliferation of cultured kidney cells, as well as the activity of HGF in accelerating regeneration of the kidney in vivo in the living body.

### Test Example 6

### Effect on the growth of human normal epidermis melanocytes

The growth accelerating action of HGF which is the active ingredient of the side effect inhibitor of the present invention, on melanocytes was confirmed in the following manner.

Human normal epidermis melanocytes (Kurabo Co., Japan) were suspended in a serum-free basic culture medium prepared by adding 5 µg/ml insulin, 0.5 µg/ml hydrocortisone, and 10 ng/ml phorbol-12-myristate-13-acetate (PMA) to MCDB 153 (high amino acid type) medium, and inoculated into a 12-well plastic plate at 10⁴ cells per well. After 24 hours' incubation at 37°C in the presence of 10% CO₂, 25% O₂ and 65% N₂, the medium was changed to a test medium prepared by adding 0 to 20 ng/ml of HGF to a serum-free basic culture medium, and the incubation was continued. On the 9th day from the initiation of the incubation, the medium was changed to a test medium containing HGF, and 15 days later, the incubation was terminated. The cells were counted by a hemocytometer.

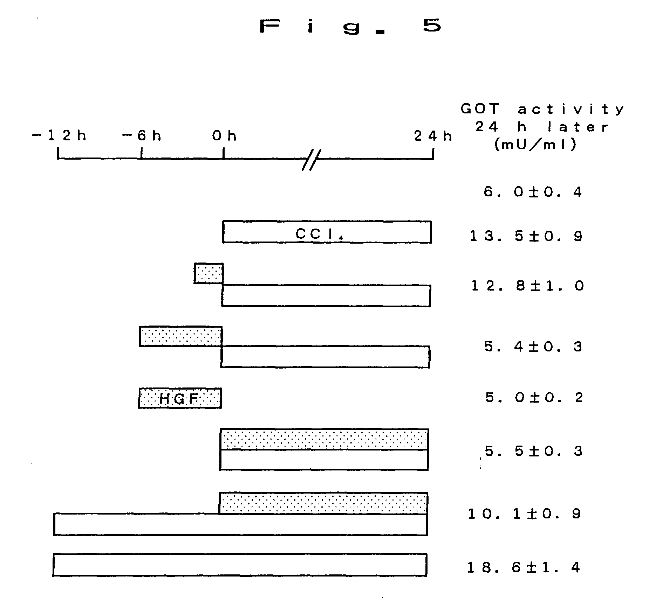

The results are shown in Fig. 7. It was confirmed that the growth of normal melanocyte was accelerated dose-dependently by HGF in the range of 0-10 ng/ml, showing about 5 times enhanced growth at the optimum concentration, as can be seen in Fig. 7.

### Test Example 7

### Effect on the synthesis of replication DNA by human normal epidermis melanocytes

Human normal epidermis melanocytes were suspended in a serum-free basic culture medium as described in Test Example 6, and the cells were inoculated into a 24-well plastic plate at 4 x 10⁴ cells/well. After 24 hours' incubation at 37°C in the presence of 10% CO₂, 25% O₂ and 65% N₂, the medium was changed to a test medium prepared by adding 0 to 20 ng/ml of HGF to the serum-free basic culture medium, and the incubation was continued. After incubation for 24 hours, 0.5 µCi of [¹²⁵I]deoxyuridine was added to each well. After the [¹²⁵I]deoxyuridine was taken into the cells by 4 hours' incubation, the cells were washed with PBS (pH 7.4), and precipitated with a cool aqueous solution of 10% trichloroacetic acid. The cells were subjected to lysis with 1 N sodium hydroxide solution, and radioactivity was measured by a gamma counter. Also, the sample which underwent radioactivity measurement was partially taken, and measured for protein amount by Micro BCA Protein Assay System (Pierce Co.). The amount of the labeled deoxyuridine which had been taken into cells was estimated by subtracting the cell count of control, and converting into per 1 mg human normal epidermis melanocyte protein, which was taken as DNA synthesis activity (dpm/mg protein).

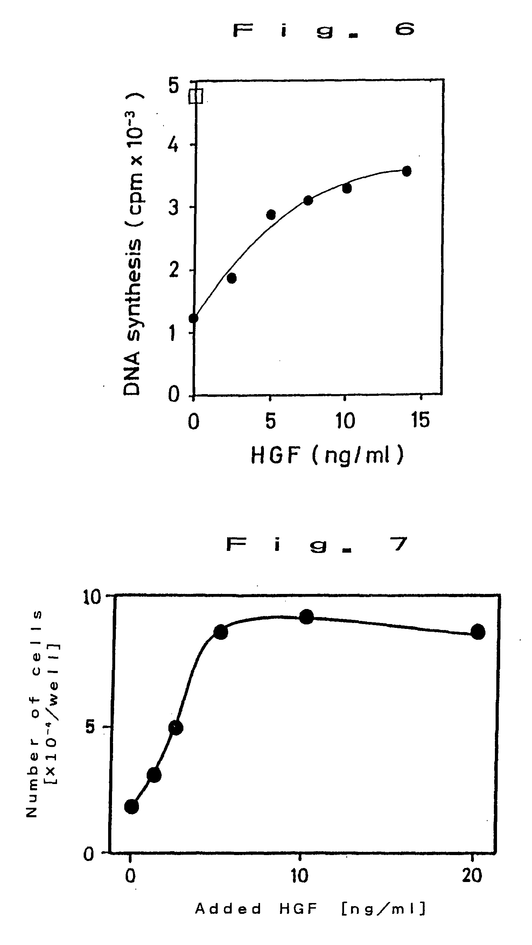

The results are shown in Fig. 8. It was confirmed that the synthesis of replication DNA by normal epidermis melanocytes was accelerated dose-dependently by HGF in the range of 0-10 ng/ml, exhibiting about 4 times enhanced synthesis at the optimum concentration, as shown in Fig. 8.

### Test Example 8

### Effect on the growth of human normal epidermis keratinocytes

The growth accelerating action of HGF which is the active ingredient of the side effect inhibitor of the present invention, on keratinocytes was confirmed in the following manner.

Human normal epidermis keratinocytes were suspended in a medium prepared by adding bovine hypothalamus extract (150 µg protein/ml) to the serum-free basic culture medium as described in Test Example 6, and seeded into a 12-well plastic plate at 10⁴ cells/well. After 24 hours' incubation at 37°C in the presence of 10% CO₂, 25% O₂ and 65% N₂, the medium was changed to a serum-free basic culture medium having a calcium ion concentration adjusted to 1.8 mM, followed by 24 hours' incubation. HGF was added thereto from 0 to 20 ng/ml, and the incubation was continued. On the 6th day from the initiation of the incubation, the medium was changed to a new medium containing HGF, and 4 days later (10th day from the initiation of the incubation), the incubation was terminated. The cells were counted by a hemocytometer.

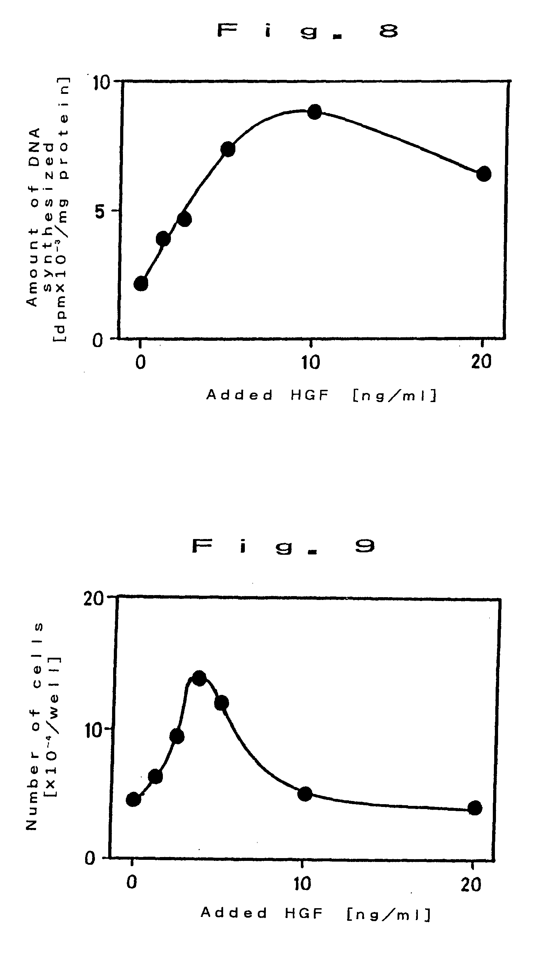

The results are shown in Fig. 9. It was confirmed that the growth of normal keratinocytes was accelerated dose-dependently by HGF in the range of 0-2.5 ng/ml, exhibiting about 3 times enhanced growth at the optimum concentration, as shown in Fig. 9.

### Test Example 9

### Effect on the synthesis of replication DNA by human normal epidermis keratinocytes

Human normal epidermis keratinocytes were suspended in a medium prepared by adding bovine hypothalamus extract (150 µg protein/ml) to the serum-free basic culture medium as described in Test Example 6, and seeded into a 24-well plastic plate at 4 x 10⁴ cells/well. After 24 hours' incubation at 37°C in the presence of 10% CO₂, 25% O₂ and 65% N₂, the medium was changed to a serum-free basic culture medium having a calcium ion concentration adjusted to 1.8 mM, followed by 24 hours' incubation. HGF was added thereto from 0 to 20 ng/ml, and the incubation was continued. After 24 hours' incubation, 0.5 µCi of [¹²⁵I]deoxyuridine was added to each well. After the [¹²⁵I]deoxyuridine was taken into the cells by 4 hours' incubation, the cells were washed twice with PBS (pH 7.4), and precipitated with a cool aqueous solution of 10% trichloroacetic acid. The cells were subjected to lysis with 1 N sodium hydroxide solution and radioactivity was measured by a gamma counter. Also, the sample which underwent radioactivity measurement was partially taken, and measured for protein amount by Micro BCA Protein Assay System (Pierce Co.). The amount of the labeled deoxyuridine which had been taken into the cells was estimated by subtracting the cell count of control, and converting into per 1 mg human normal epidermis keratinocyte protein, which was taken as DNA synthesis activity (dpm/mg protein).

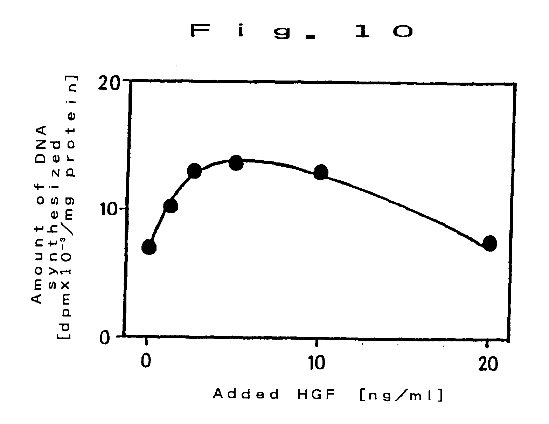

The results are shown in Fig. 10. It was confirmed that the synthesis of replication DNA by normal epidermis keratinocytes was accelerated dose-dependently by HGF in the range of 0-5 ng/ml, exhibiting about 2 times enhanced synthesis at the optimum concentration, as shown in Fig. 10.

### Example 1

A solution was aseptically prepared by adding 100 mg of HGF, 1 g of mannitol, and 10 mg of Polysorbate 80 to 100 ml of physiological saline, then the solution was aseptically filled in a vail at 1 ml per vial. The contents of the vials were freeze-dried according to the conventional method to obtain the HGF product in the freeze-dried form.

### Example 2

An aqueous solution was aseptically prepared by adding 100 mg of HGF and 100 mg of human serum albumin to 100 ml of a 0.02 M phosphate buffer at pH 7.4 containing 0.15 M NaCl and 0.01% Polysorbate 80. The solution was aseptically filled in a vial at 1 ml per vial. The contents of the vials were freeze-dried according to the conventional method to obtain the HGF product in the freeze-dried form.

### Industrial Applicability

The medicament for preventing side effects of cancer therapy of the invention contains the effective component of HGF. This HGF can reduce damage inflicted on the normal cells and tissues by chemotherapy and radiation therapy. By this property, the medicament ameliorates side effects in cancer therapy so that it is possible to perform more potent therapy, resulting in an improved anti-cancer effect, which is extremely useful in clinical practice.

HGF carries out the effect of accelerating proliferation only on the normal epithelial cells without any effect on the proliferation of normal interstitial cells such as nonparenchymal cells of the liver or fibroblasts. HGF is free of activity in inducing carcinogenesis in cells, so one can conclude that the medicament of this invention possesses a high specificity and does not have side effects.

## Claims

1. Use of hepatocyte growth factor (HGF) for preparing a medicament for preventing side effects of cancer therapy in humans or other mammals.

2. The use of claim 1, wherein the medicament further comprises an acceptable carrier.

3. The use of claim 1 or 2, wherein the HGF is derived from tissues or blood components of humans or other animals.

4. The use of claim 1 or 2, wherein the HGF is produced by the method of gene recombination.

5. The use of claim 4, wherein the host for gene recombination is E. coli, B. subtilis, yeasts, filamentous fungi, or plant or animal cells.

## Patentansprüche

1. Verwendung von Hepatocytenwachstumsfaktor (HGF) zur Herstellung eines Medikaments zur Prävention von Nebenwirkungen der Krebstherapie bei Menschen oder anderen Säugern.

2. Verwendung gemäß Anspruch 1, wobei das Medikament weiterhin einen annehmbaren Träger umfasst.

3. Verwendung gemäß Anspruch 1 oder 2, wobei der HGF von Geweben oder Blutkomponenten von Menschen oder anderen Tieren abgeleitet ist.

4. Verwendung gemäß Anspruch 1 oder 2, wobei der HGF nach dem Verfahren der Genrekombination hergestellt ist.

5. Verwendung gemäß Anspruch 4, wobei es sich bei dem Wirt für die Gen-Rekombination um *E. coli, B. subtilis*, Hefen, Fadenpilze oder Pflanzen - oder Tierzellen handelt.

## Revendications

1. Utilisation d'un facteur de croissance d'hépatocytes (HGF) pour préparer un médicament afin d'empêcher les effets secondaires de la thérapie du cancer chez les humains ou d'autres mammifères.

2. Utilisation selon la revendication 1, dans laquelle le médicament comprend en outre un véhicule acceptable.

3. Utilisation selon la revendication 1 ou 2, dans laquelle le HGF est tiré de tissus ou de composants sanguins d'humains ou d'autres animaux.

4. Utilisation selon la revendication 1 ou 2, dans laquelle le HGF est produit par le procédé de recombinaison de gènes.

5. Utilisation selon la revendication 4, dans laquelle l'hôte utilisé pour la recombinaison de gènes est la souche E. coli, la souche B. subtilis, les levures, les champignons filamenteux ou des cellules végétales ou animales.
